# EUROPÄISCHE PATENTSCHRIFT

(11) **EP 2 904 988 B1**
(45) Veröffentlichungstag und Bekanntmachung des Hinweises auf die Patenterteilung: **01.04.2020**
(21) Anmeldenummer: 14153942.9
(22) Anmeldetag: 05.02.2014
(51) Int. Cl.: A61C 9/00, A61B 5/00, G01B 11/25, G06T 7/00

(54) **Verfahren zur intraoralen dreidimensionalen Vermessung**
Method for intraoral three-dimensional measurement
Procédé de mesure tridimensionnelle intraorale

(43) Veröffentlichungstag der Anmeldung: 12.08.2015
(73) Patentinhaber: Sirona Dental Systems GmbH, 64625 Bensheim (DE)
(72) Erfinder: Thiel, Frank, 64372 Ober-Ramstadt (DE); Pfeiffer, Joachim, 64625 Bensheim (DE)
(74) Vertreter: Özer, Alpdeniz

(56) Entgegenhaltungen:
- EP-A1- 1 277 432
- EP-A1- 1 716 816
- DE-A1-102012 100 953
- US-A- 4 837 732
- US-A1- 2010 158 490

## Beschreibung

### Technisches Gebiet

Die Erfindung betrifft ein Verfahren zur optischen dreidimensionalen Vermessung mit einer intraoralen Kamera, wobei die Kamera in schneller zeitlicher Abfolge 2D-Aufnahmen erzeugt und wobei aus mindestens einer 2D-Aufnahme der Vermessungskamera ein 3D-Bild erzeugt wird. In einem ersten Betriebsmodus wird ein 3D-Gesamtbild aus einer Vielzahl aller erzeugten 2D-Aufnahmen bzw. erzeugten Sequenzen erzeugt.

### Stand der Technik

Es sind verschiedene Arten von intraoralen 3D-Messkameras bekannt.

Eine erste Art von intraoralen 3D-Messkameras erzeugt ein 3D-Bild auf Basis mehrerer Aufnahmen des Objekts aus gleicher Perspektive. Diese Art von Kameras muss also für ein gewisses Zeitintervall, welches für das Detektieren der mehreren Aufnahmen benötigt wird, möglichst ruhig gehalten werden.

Dies stellt an einen Benutzer hohe Anforderungen. Die Gefahr, verwackelte und damit unbrauchbare Aufnahmen zu erzeugen bzw. die Vermessung sogar mehrfach wiederholen zu müssen, besteht.

Eine zweite Art von intraoralen 3D-Messkameras erzeugt ein 3D-Bild bereits anhand einer einzigen Aufnahme. Ein Stillhalten der Kamera ist entsprechend zumindest dann nicht nötig, wenn die einzige Aufnahme schnell genug, beispielsweise in weniger als einer Sekunde, durchgeführt wird. Dies ermöglicht das Aufnehmen unter Bewegung und damit das kontinuierliche Vermessen von Bereichen, die größer sind, als das Sichtfeld der Kamera. Hierfür wird die Kamera langsam über den zu vermessenden Bereich bewegt und es werden kontinuierlich einzelne Aufnahmen erzeugt. Aus jeder einzelnen Aufnahme kann dann ein 3D-Bild erzeugt werden und die erzeugten 3D-Bilder können zu einem dreidimensionalen Bild des gesamten Bereichs zusammengesetzt werden.

Eine dritte Art von intraoralen 3D-Messkameras erzeugt ein 3D-Bild aus einer Sequenz mehrerer Aufnahmen, wie die beschriebene erste Art, jedoch ist die Aufnahmefrequenz so hoch, dass das Aufnehmen einer Sequenz beispielsweise deutlich weniger als 1s dauert. Weiterhin können Effekte, die auf Grund einer Bewegung der Kamera auftreten, beispielsweise rechnerisch bei der Erzeugung einer 3D-Aufnahme aus den mehreren Aufnahmen einer Sequenz in ihrer Wirkung neutralisiert werden. Hierdurch ist es möglich 3D-Messkameras der dritten Art ebenfalls wie manche Kameras der zweiten Art für kontinuierliche Aufnahmen unter Bewegen der Kamera bzw. für das kontinuierliche Vermessen größerer Bereiche zu verwenden.

Auch der kontinuierliche Aufnahmemodus zur Vermessung größerer Bereich durch ein kontinuierliches Bewegen der Kamera über den zu vermessenden Bereich, den intraorale 3D-Messkameras der zweiten und dritten Art bereitstellen, stellt hohe Anforderungen an den Benutzer. Es sollte beispielsweise vermieden werden, bewegliche Bereiche, wie die Zunge, die Wangen oder die Lippen mit aufzunehmen, da diese, wenn sie zwischen verschiedenen Aufnahmen bzw. Aufnahmesequenzen bewegt werden, ein Zusammenfügen der einzelnen 3D-Aufnahmen erschweren, verfälschen oder gar verhindern können. Auch die Kontrolle des erzeugten Datenvolumens ist für den Benutzer schwerer zu kontrollieren, wobei ein hohes Datenaufkommen, also unnötig viele erzeugte Aufnahmen, zu Verzögerungen bei der Erzeugung der 3D-Aufnahmen bzw. der 3D-Gesamtaufnahem führen können.

Das Dokument US 2010/0158490 A1 beschreibt ein Verfahren zur optischen dreidimensionalen Vermessung mittels einer intraoralen Kamera, wobei eine Sequenz von 2D-Bildern anhand einer zu den Bildern berechneten Verwacklungszahl ausgewählt wird, um aus den ausgewählten Bildern ein 3D-Bild zu erzeugen.

Die Aufgabe der vorliegenden Erfindung ist es daher, ein Verfahren zur Verfügung zu stellen, welches das Aufnehmen insbesondere von größeren Bereichen auf möglichst einfache und zuverlässige Weise ermöglicht.

### Offenbarung der Erfindung

Diese Aufgabe wird durch ein Verfahren zur optischen dreidimensionalen Vermessung mit einer intraoralen Kamera gelöst, wobei die intraorale Kamera in zeitlicher Abfolge einzelne 2D-Aufnahmen oder Sequenzen von mehreren aufeinanderfolgend erzeugten 2D-Aufnahmen erzeugt und wobei aus mindestens einer der einzelnen 2D-Aufnahmen oder aus mindestens einer der Sequenzen ein 3D-Bild erzeugt wird. Es wird zwischen einem ersten und einem zweiten Betriebsmodus gewählt. Im ersten Betriebsmodus wird eine Vielzahl aller erzeugten einzelnen 2D-Aufnahmen bzw. erzeugten Sequenzen zur Erzeugung eines 3D-Gesamtbilds verwendet. Im zweiten Betriebsmodus wird eine Vielzahl aller während eines Zeitintervalls erzeugten 2D-Aufnahmen bzw. erzeugten Sequenzen zur Erzeugung eines 3D-Gesamtbilds verwendet, wobei das Zeitintervall direkt vor und/oder nach einem Auslösebefehl liegt. Das erfindungsgemäße Verfahren ist in den Ansprüchen 1 bis 10 definiert.

Je nach Aufnahmeart kann ein 3D-Bild aus mindestens einem 2D-Bild oder auch aus mindestens einer Sequenz von 2D-Bildern erzeugt werden. Das als 3D-Gesamtbild bezeichnete Messergebnis kann aus mindestens einem solchen 3D-Bild erzeugt bzw. zusammengesetzt werden. Es ist aber auch möglich das 3D-Gesamtbild direkt aus den 2D-Aufnahmen, also aus mindestens einer 2D-Aufnahme oder mindestens einer Sequenz von 2D-Aufnahmen zu erzeugen.

Die zwei Betriebsmodi ermöglichen es dem Benutzer zwischen einer Art ständigem 3D-Video-Betrieb und einer Art "point-and-click" Betrieb zu wählen.

Im ersten Betriebsmodus, also dem 3D-Video-Betrieb, läuft die Aufnahme kontinuierlich, beispielsweise vom in Betrieb nehmen der Kamera bis zu ihrem Ausschalten. Es werden kontinuierlich 2D-Aufnahmen oder Sequenzen erzeugt und zu jeder 2D-Aufnahme bzw. zu jeder Sequenz ein 3D-Bild erzeugt. Die erzeugten 3D-Bilder können dann kontinuierlich nach und nach oder nach Ende der gesamten Aufnahmeprozedur zu einem 3D-Gesamtbild zusammengesetzt werden.

Der zweite Betriebsmodus, der "point-and-click" Betrieb, sieht dahingehend vor, dass zwar kontinuierlich 2D-Aufnahmen erzeugt, aber erst nach einem Auslösebefehl 2D-Aufnahmen auch weiter verarbeitet werden. Dies ermöglicht es beispielsweise dem Benutzer, die Kamera erst in aller Ruhe über dem aufzunehmenden Objekt zu positionieren, ehe dessen Vermessung beginnt und aus den dann kontinuierlich erzeugten 2D-Aufnahmen bzw. Sequenzen jeweils 3D-Bilder erzeugt und nach und nach oder nach Abschluss der gesamten Vermessung bzw. Ablauf des Zeitintervalls zu einem 3D-Gesamtbild zusammengesetzt werden. Bei entsprechend kurzer Wahl kann hierdurch auch ein bloßes dreidimensionales Fotografieren eines anvisierten Bereichs des Objekts erreicht werden, indem während des Zeitabschnitts mindestens eine 2D-Aufnahme oder mindestens eine Sequenz von 2D-Aufnahmen zur Berechnung genau eines 3D-Bilds aufgenommen wird und das daraus berechnete 3D-Bild als 3D-Gesamtbild abgespeichert wird. Das als Ergebnis der Vermessung präsentierte 3D-Gesamtbild kann somit aus einem oder aus mehreren 3D-Bildern zusammengesetzt sein. Der zweite Betriebsmodus ermöglicht es somit, sowohl den aufgenommenen Bildausschnitt, als auch das Datenaufkommen besser zu kontrollieren.

Um das Positionieren zu erleichtern, können beispielsweise die erzeugten 2D-Aufnahmen oder auch mit der Kamera zwischen zwei einzelnen 2D-Aufnahmen oder zwischen zwei Sequenzen erzeugte weitere 2D-Aufnahmen dem Benutzer beispielsweise auf einem Bildschirm angezeigt werden. Solche Preview-Funktionen sind bereits bekannt. Je nach Vermessungstechnik können diese weiteren 2D-Aufnahmen beispielsweise nach dem Wegklappen eines zur Projektion von Mustern in einem Beleuchtungsstrahl angeordneten Gitters oder mit einem sehr schnell senkrecht zu einem Beleuchtungsstrahl bewegten Gitter erzeugt werden.

Vorteilhafterweise wird der Auslösebefehl durch das Betätigen eines Fußschalters erzeugt.

Ein Fußschalter ermöglicht es, kontrolliert den Auslösebefehl auszulösen, ohne dass hierdurch die möglichst kontrollierte Bewegung der handgehaltenen Kamera gestört wird.

Vorteilhafterweise wird der Auslösebefehl durch einen Bewegungssensor erzeugt, wenn dieser ein Stillhalten der Vermessungskamera detektiert.

Hierdurch können wackelnde und/oder ruckartige Bewegungen der Kamera vermieden werden, die durch das Betätigen eines Schalters mit dem Fuß oder auch mit einem freien Finger, einer freien Hand verursacht werden.

Die Kamera kann hierbei ganz unbeschwert bewegt und über der gewünschten Szene positioniert werden, ohne dass Daten erzeugt werden. Hat der Benutzer die gewünschte Szene gefunden bzw. die Kamera in der gewünschten Position positioniert, so muss er die Kamera lediglich kurz still halten, um die Vermessung auszulösen und die Kamera im Anschluss beispielsweise kontrolliert über einen zu vermessenden Bereich zu bewegen.

Vorteilhafterweise wird während des Aufnehmens aus jeweils mindestens zwei aufeinanderfolgend erzeugten 2D-Aufnahmen eine Verwacklungszahl bestimmt und mit einem Grenzwert verglichen, wobei bei unterschreiten des Grenzwerts ein Auslösebefehl erzeugt wird.

Das Bestimmen einer Verwacklungszahl aus den erzeugten 2D-Aufnahmen selbst ermöglicht es, den Auslösebefehl durch das Stillhalten der Kamera auszulösen, ohne dass ein weiteres Bauteil, wie ein Sensor notwendig ist.

Vorteilhafterweise werden die einzelnen 2D-Aufnahmen oder Sequenzen nacheinander in einem Zwischenspeicher abgelegt, wobei eine Anzahl von einzelnen 2D-Aufnahmen bzw. Sequenzen abgelegt wird und nach einem Erreichen der Anzahl bereits abgelegte einzelne 2D-Aufnahmen bzw. Sequenzen durch neue einzelne 2D-Aufnahmen bzw. Sequenzen überschrieben werden.

Das zumindest vorübergehende Ablegen der 2D-Aufnahmen in einem Zwischenspeicher ermöglicht beispielsweise eine relative freie Wahl des Verhältnisses von Aufnahmezeitintervall und Auslösebefehl. Durch das Überschreiben können die Anforderungen an die Größe des Zwischenspeichers reduziert werden. Ein solcher Zwischenspeicher kann beispielsweise direkt in der handgehaltenen Kamera angeordnet sein. Unter Speicher oder Zwischenspeicher wird hier jedes Medium verstanden, welches Bild-Daten zumindest eines Bildes aufnehmen und aus welchem Bild-Daten mindestens eines Bildes ausgelesen werden können.

Vorteilhafterweise werden nach dem Auslösebefehl alle innerhalb eines dem Auslösebefehl vorangegangenen Zeitintervalls oder alle innerhalb eines sich dem Auslösebefehl anschließenden Zeitintervalls oder alle innerhalb eines einen Zeitpunkt des Auslösebefehls umfassenden Zeitintervalls erzeugten 2D-Aufnahmen in einen Speicher übertragen.

Zur weiteren Verarbeitung bzw. zur Dokumentation werden die Daten für die 3D-Bilder bzw. das 3D-Gesamtbild in einem Speicher abgelegt. Wird die Kamera im zweiten Betriebsmodus betrieben, so kann das Datenaufkommen dadurch kontrolliert werden, dass nur die Daten im Aufnahmezeitintervall beispielsweise direkt oder aus einem Zwischenspeicher in den Speicher übertragen werden. Das Aufnahmezeitintervall wird durch den Auslösebefehl bestimmt. Es kann entweder ein Zeitintervall sein, welches sich direkt an den Zeitpunkt der Erzeugung des Auslösebefehls anschließt. Es kann aber auch ein um den Auslösebefehl herum liegendes Zeitintervall oder sogar ein dem Zeitpunkt der Erzeugung des Auslösebefehls direkt vorangehendes Zeitintervall sein. Der Speicher kann beispielsweise in der Kamera selbst oder in einer Recheneinheit oder ähnlichem angeordnet sein, wobei die Daten beispielsweise mittels eines Kabels oder auch kabellos übertragen werden können.

Vorteilhafterweise werden die 2D-Aufnahmen oder zusätzlich erzeugte 2D-Aufnahmen auf einem Bildschirm nacheinander angezeigt.

Dies kann die Orientierung bzw. Positionierung der Kamera erleichtern. Es kann auch eine Kontrolle der Position der Kamera während der Vermessung ermöglichen.

Vorteilhafterweise wird das mindestens eine 3D-Bild und/oder das 3D-Gesamtbild auf einem Bildschirm angezeigt.

Werden die 3D-Bilder, die aus erzeugten 2D-Aufnahmen erzeugt werden, direkt nacheinander angezeigt, so kann die Position der Kamera während der Vermessung anhand dieser kontrolliert werden. Es ist auch möglich, die erzeugten 3D-Bilder kontinuierlich zusammenzufügen, so dass der Benutzer direkt das Ergebnis bzw. Zwischenergebnis seiner Vermessung, nämlich das Entstehen des 3D-Gesamtbilds kontrollieren kann.

Vorteilhafterweise hat das Zeitintervall eine vorbestimmte oder vorab bestimmbare Dauer.

Hierdurch wird das Datenaufkommen begrenzt. Das Zeitintervall kann beispielsweise vorab eingestellt oder ausgewählt werden. Das Ende kann dem Benutzer beispielsweise an einem Bildschirm oder durch eine Lampe oder ähnliches an der Kamera oder auch durch einen Ton angezeigt werden. Vorteilhafterweise endet das Zeitintervall nach einem Stop-Befehl.

Hierdurch kann der Benutzer die Aufnahmedauer an seine Bedürfnisse anpassen, wodurch insbesondere das Datenaufkommen minimiert werden kann. Der Stop-Befehl kann beispielsweise durch einen Schalter an der Kamera, durch einen Fußschalter oder auch mittels eines verbalen Befehls und eines entsprechenden akustischen Sensors erzeugt werden. Besonders einfach ist es, den Stop-Befehl in gleicher Weise wie den Auslösebefehl zu erzeugen. Wird beispielsweise der Auslösebefehl mittels eines Fußschalters erzeugt, so kann das darauffolgende Betätigen des Fußschalters den Stop-Befehl erzeugen.

Weiterhin wird eine Vorrichtung zur intraoralen dreidimensionalen optischen Vermessung beschrieben, die eine handgehaltene intraorale Kamera und mindestens einen innerhalb oder außerhalb der Kamera angeordneten Speicher aufweist. Mit der Vorrichtung sind kontinuierlich 2D-Aufnahmen erzeugbar. Weiterhin weist die Vorrichtung ein Mittel zum Auswählen eines ersten oder eines zweiten Betriebsmodus sowie eine Auslöseeinheit auf.

Die Erweiterung einer intraoralen Kamera für kontinuierliche Messungen, auch on-the-fly Messungen genannt, um eine Auslöseeinheit und Wahlmittel hinsichtlich des Betriebsmodus ermöglichen es einem Benutzer, das Datenaufkommen und den Bildausschnitt einfacher kontrollieren zu können.

Vorteilhafterweise ist die Auslöseeinheit ein Fußschalter, der ein besonders einfaches Mittel zur Erzeugung eines Auslösebefehls darstellt.

Vorteilhafterweise umfasst die Auslöseeinheit einen an der Vermessungskamera angeordneten Bewegungssensor und eine Vergleichseinheit, wobei mit dem Bewegungssensor ein Bewegungswert erzeugbar ist, der mittels der Vergleichseinheit mit einem voreinstellbaren Vergleichswert vergleichbar ist.

Hierdurch kann auf einfache Weise das Stillhalten der Kamera detektiert werden.

Vorteilhafterweise weist die Auslöseeinheit eine Verwacklungsanalyseeinheit zur Bestimmung einer Verwacklungszahl aus mindestens zwei erzeugten Aufnahmen auf.

Eine Verwacklungsanalyseeinheit kann das Stillhalten anhand zweier erzeugter Aufnahmen ermitteln. Es ist somit kein weitere Sensor mehr notwendig.

Vorteilhafterweise umfasst die Vorrichtung einen Bildschirm, auf dem mit der Vermessungskamera erzeugte Aufnahmen nebeneinander und/oder nacheinander anzeigbar sind. Hierdurch kann die Positionierung bzw. die Führung der Kamera einfach kontrolliert werden.

Vorteilhafterweise weist die Vorrichtung eine Stopeinheit auf. Diese ermöglicht es dem Benutzer, die Aufnahmedauer zu kontrollieren. Besonders einfach ist es, wenn die Stopeinheit mit der Auslöseeinheit identisch ist, wenn also die Auslöseeinheit auch als Stopeinheit fungiert.

### Kurze Beschreibung der Zeichnungen

Das erfindungsgemäße Verfahren wird anhand der Zeichnung erläutert. Es zeigen:
- Fig. 1: eine Vorrichtung zur intraoralen dreidimensionalen optischen Vermessung,
- Fig. 2: eine Vorrichtung mit einer als Verwacklungsanalyseeinheit ausgebildeten Auslöseeinheit,
- Fig. 3: ein Zeitintervall im Verhältnis zu einem Auslösbefehl,
- Fig. 4: ein durch einen Auslösebefehl und einen Stop-Befehl definiertes Zeitintervall.

### Ausführungsformen

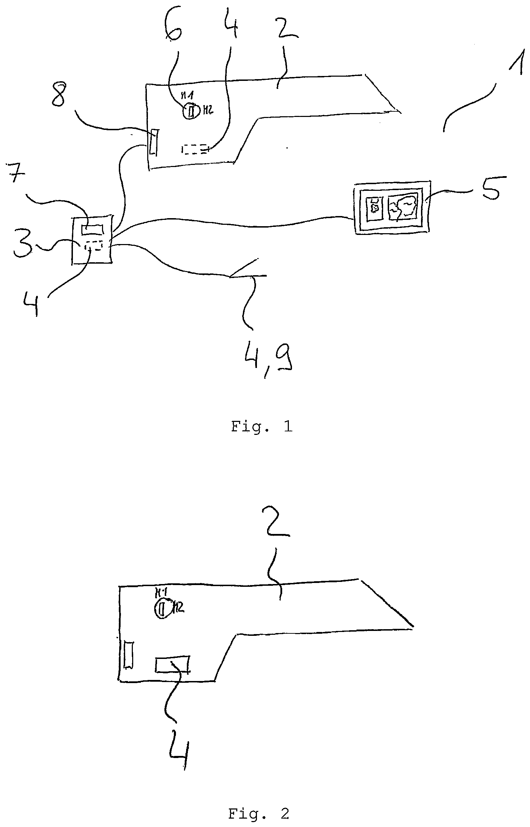

In Fig. 1 ist eine Vorrichtung 1 zur intraoralen dreidimensionalen optischen Vermessung skizziert, die eine handgehaltene intraorale Kamera 2, eine Recheneinheit 3, eine als Fußschalter ausgebildete Auslöseeinheit 4 und ein als Bildschirm ausgebildetes Anzeigemittel 5 umfasst. Die intraorale Kamera 2 weist einen Schalter als Mittel 6 zum Auswählen eines Betriebsmodus M1, M2 auf.

Die Kamera 2 kann beispielsweise eine auf Triangulation beruhende Vermessungseinheit sein, die ein aufzunehmendes Objekt mittels darauf projizierter Muster vermisst. Der Aufnahmevorgang, also beispielsweise das Aufprojizieren des Musters und das Detektieren des zurückgestreuten Lichts als 2D-Aufnahme, wird beispielsweise gestartet, sobald die Kamera aus einer Halterung genommen oder eingeschaltet wird. Die Kamera 2 detektiert dann kontinuierlich 2D-Aufnahmen.

Je nach Vermessungsmethode kann aus einer einzelnen 2D-Aufnahme oder aus einer Sequenz aus mehreren aufeinanderfolgend erzeugten 2D-Aufnahmen jeweils ein 3D-Bild erzeugt werden. Alle erzeugten 3D-Bilder können entweder nach Abschluss des Aufnahmevorgangs oder auch nach und nach während des Aufnahmevorgangs zu einem 3D-Gesamtbild zusammengefügt werden.

Statt als Fußschalter kann die Auslöseeinheit 4 beispielsweise auch als ein an der intraoralen Kamera 2 angeordneter Bewegungssensor und eine Vergleichseinheit ausgebildet sein, wie dies in Fig. 1 mit gestrichelter Linie skizziert ist. Die Vergleichseinheit kann entweder, wie in Fig. 1 gestrichelt dargestellt, in der Recheneinheit 3 oder auch in der Kamera 2 angeordnet werden.

Weiterhin ist es möglich, die Auslöseeinheit 4 als Verwacklungsanalyseeinheit auszubilden, die beispielsweise in der intraoralen Kamera 2 angeordnet sein kann, wie es in Fig. 2 dargestellt ist. Die Verwacklungsanalyseeinheit kann aber auch in der Recheneinheit 3 angeordnet bzw. ein Teil der Recheneinheit 3 sein. Aus mindestens zwei aufeinanderfolgend erzeugten 2D-Aufnahmen wird mittels der Verwacklungsanalyseeinheit eine Verwacklungszahl V bestimmt und mit einem Grenzwert G verglichen. Ein Auslösebefehl A wird von der als Verwacklungsanalyseeinheit ausgebildeten Auslöseeinheit 4 dann ausgegeben, wenn die ermittelte Verwacklungszahl V kleiner als der Grenzwert G ist.

Das erfindungsgemäße Verfahren sieht vor, dass ein Benutzer beispielsweise mittels eines als Schalter ausgebildeten Mittels 6 zwischen einem ersten und einem zweiten Betriebsmodus M1, M2 auswählen kann.

Im ersten Betriebsmodus M1 werden die kontinuierlich erzeugten bzw. detektierten 2D-Aufnahmen in einen beispielsweise in der Recheneinheit 3 angeordneten Speicher 7 übertragen. Der Speicher 7 könnte auch in der Kamera 2 selbst angeordnet sein, wenn er ausreichend klein und leicht ausgebildet ist. Zumindest aus einer Vielzahl aller in den Speicher 7 übertragenen 2D-Aufnahmen wird ein 3D-Gesamtbild erzeugt, wobei hierfür jeweils aus einer einzelnen 2D-Aufnahme oder aus einer Sequenz ein 3D-Bild errechnet wird und die errechneten 3D-Bilder zusammengefügt werden. Einige der übertragenen 2D-Aufnahmen können beispielsweise aufgrund schlechter Qualität bewusst verworfen werden, beispielsweise bei niedriger Datenpunkteanzahl.

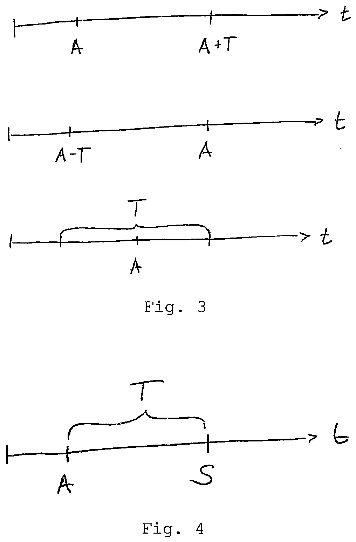

Im zweiten Betriebsmodus M2 werden erst auf einen Auslösebefehl A hin 2D-Aufnahmen in den Speicher 7 übertragen und zwar alle während eines sich nach einem Zeitpunkt des Auslösebefehls A richtenden Zeitintervalls T detektierten 2D-Aufnahmen. Zumindest eine Vielzahl dieser in den Speicher 7 übertragenen 2D-Aufnahmen wird für das 3D-Gesamtbild verwendet. Einige 3D-Aufnahmen können beispielsweise aufgrund einer schlechten Qualität verworfen werden. Das Zeitintervall T kann sich beispielsweise direkt an den Zeitpunkt des Auslösebefehls anschließen, wie dies in Fig. 3 oben skizziert ist. Das Zeitintervall T kann auch dem Zeitpunkt des Auslösebefehls A direkt vorangestellt sein, wie dies in Fig. 3 in der Mitte dargestellt ist. Alternativ kann das Zeitintervalls T auch den Zeitpunkt des Auslösebefehls A umfassen, wie es in Fig. 3 unten skizziert ist.

Im Falle der letzten beiden Varianten ist es notwendig, einen Zwischenspeicher 8 beispielsweise innerhalb der Kamera 2 oder in der Recheneinheit 3 anzuordnen oder den Speicher 7 auch als Zwischenspeicher einzusetzen und in diesem Zwischenspeicher 8 immer die aktuellsten 2D-Aufnahmen abzulegen. Der Zwischenspeicher 8 muss entsprechend ausreichend groß sein, dass alle bereits erzeugten, gewünschten 2D-Aufnahmen je nach Wahl des Zeitintervalls T relativ zum Zeitpunkt des Auslösebefehls A noch vorhanden sind und in den Speicher 7 übertragen werden können.

Das Zeitintervall T, also dessen Dauer, kann beispielsweise vorab festgelegt oder ausgewählt werden. Es ist auch möglich, das Zeitintervall durch einen Stopbefehl S zu beenden. Wie in Fig. 4 skizziert, kann das Zeitintervall beispielsweise mit dem Auslösebefehl A beginnen und mit dem Stopbefehl S enden.

Hierfür kann die Vorrichtung 1 eine Stopeinheit 9 aufweisen. Diese kann beispielsweise als Fußschalter ausgebildet und/oder mit der Auslöseeinheit 4 identisch sein, wie es in Fig. 1 dargestellt ist.

Unabhängig vom Betriebsmodus M1, M2 können die erzeugten 2D-Aufnahmen oder zusätzlich erzeugte 2D-Aufnahmen auf einem Anzeigemittel 5 nacheinander angezeigt werden, so dass dem Benutzer eine Art 2D-Video zu Orientierungszwecken bereitgestellt wird. Weiterhin ist es möglich, alle bereits erzeugten 3D-Bilder auf einem Anzeigemittel 5, beispielsweise gleichzeitig mit den 2D-Aufnahmen, anzuzeigen. Die 3D-Bilder können nacheinander angezeigt werden, so dass eine Art 3D-Video entsteht. Es ist auch möglich, die 3D-Bilder nach und nach zusammenzufügen und immer das bereits zusammengefügte 3D-Gesamtbild anzuzeigen, so dass der Benutzer schon während des Aufnahmevorgangs der Entstehung der 3D-Gesamtbilds zusehen kann.

### Bezugszeichen

- 1: Vorrichtung zur intraoralen dreidimensionalen optischen Vermessung
- 2: intraorale Kamera
- 3: Recheneinheit
- 4: Auslöseeinheit
- 5: Anzeigemittel
- 6: Mittel zum Auswählen eines Betriebsmodus
- 7: Speicher
- 8: Zwischenspeicher
- A: Auslösebefehl
- G: Grenzwert
- M1: erster Betriebsmodus
- M2: zweiter Betriebsmodus
- S: Stopbefehl
- V: Verwacklungszahl

## Patentansprüche

1. Verfahren zur optischen dreidimensionalen Vermessung mit einer intraoralen Kamera (2),
- wobei die intraorale Kamera (2) in zeitlicher Abfolge kontinuierlich einzelne 2D-Aufnahmen oder Sequenzen von mehreren aufeinanderfolgend erzeugten 2D-Aufnahmen erzeugt,
- wobei aus mindestens einer der 2D-Aufnahmen oder aus mindestens einer der Sequenzen ein 3D-Bild erzeugt wird und
- wobei in einem ersten Betriebsmodus (M1) zu einer Vielzahl aller erzeugten 2D-Aufnahmen bzw. erzeugten Sequenzen jeweils ein 3D-Bild erzeugt wird und die Vielzahl der erzeugten 2D-Aufnahmen bzw. erzeugten Sequenzen oder die erzeugten 3D-Bilder zur Erzeugung eines 3D-Gesamtbilds verwendet werden,
**dadurch gekennzeichnet, dass**
zwischen dem ersten und einem zweiten Betriebsmodus (M1, M2) gewählt wird,
- wobei im zweiten Betriebsmodus (M2) nur zu einer Vielzahl aller während eines Zeitintervalls (T) direkt vor und/oder nach einem Auslösebefehl (A) erzeugten 2D-Aufnahmen bzw. erzeugten Sequenzen ein 3D-Bild erzeugt wird und wobei die Vielzahl der erzeugten 2D-Aufnahmen bzw. erzeugten Sequenzen oder die erzeugten 3D-Bilder zur Erzeugung eines 3D-Gesamtbilds verwendet werden.

2. Verfahren nach Anspruch 1, **dadurch gekennzeichnet, dass** der Auslösebefehl (A) durch das Betätigen eines Fußschalters erzeugt wird.

3. Verfahren nach Anspruch 1, **dadurch gekennzeichnet, dass** der Auslösebefehl (A) durch einen Bewegungssensor erzeugt wird, wenn dieser ein Stillhalten der Vermessungskamera detektiert.

4. Verfahren nach Anspruch 1, **dadurch gekennzeichnet, dass** während des Aufnehmens aus jeweils mindestens zwei aufeinanderfolgend erzeugten 2D-Aufnahmen eine Verwacklungszahl (V) bestimmt und mit einem Grenzwert (G) verglichen wird und dass bei unterschreiten des Grenzwerts (G) ein Auslösebefehl (A) erzeugt wird.

5. Verfahren nach einem der Ansprüche 1 bis 4, **dadurch gekennzeichnet, dass** die 2D-Aufnahmen oder die Sequenzen von 2D-Aufnahmen nacheinander in einem Zwischenspeicher (8) abgelegt werden, wobei eine Anzahl von 2D-Aufnahmen bzw. Sequenzen abgelegt wird und nach einem Erreichen der Anzahl bereits abgelegte 2D-Aufnahmen bzw. Sequenzen durch neue 2D-Aufnahmen bzw. Sequenzen überschrieben werden.

6. Verfahren nach einem der Ansprüche 1 bis 5, **dadurch gekennzeichnet, dass** nach dem Auslösebefehl (A) alle innerhalb eines dem Auslösebefehl (A) vorangegangenen Zeitintervalls (T) oder alle innerhalb eines sich dem Auslösebefehl (A) anschließenden Zeitintervalls (T) oder alle innerhalb eines einen Zeitpunkt des Auslösebefehls (A) umfassenden Zeitintervalls (A) erzeugten 2D-Aufnahmen oder Sequenzen mehrerer 2D-Aufnahemn in einen Speicher übertragen werden.

7. Verfahren nach einem der Ansprüche 1 bis 6, **dadurch gekennzeichnet, dass** die 2D-Aufnahmen oder zusätzlich erzeugte 2D-Aufnahmen auf einem Anzeigemittel (5) nacheinander angezeigt werden.

8. Verfahren nach einem der Ansprüche 1 bis 7, **dadurch gekennzeichnet, dass** die mindestens eine 3D-Aufnahme auf einem Anzeigemittel (5) angezeigt wird.

9. Verfahren nach einem der Ansprüche 1 bis 8, **dadurch gekennzeichnet, dass** das Zeitintervall (T) eine vorbestimmte oder vorab bestimmbare Dauer hat.

10. Verfahren nach einem der Ansprüche 1 bis 8, **dadurch gekennzeichnet, dass** das Zeitintervall (T) mit dem Auslösebefehl (A) beginnt und nach einem Stop-Befehl (S) endet.

## Claims

1. A method for optical three-dimensional measurement with an intraoral camera (2),
- the intraoral camera (2) continuously generating individual 2D images or sequences of a plurality of successively generated 2D images in chronological order,
- a 3D picture being generated from at least one of the 2D images or from at least one of the sequences and
- a 3D picture being generated in a first operating mode (M1) for a plurality of all generated 2D images or generated sequences and the large number of generated 2D images or generated sequences or the generated 3D pictures being used for generating a general 3D picture,
**characterised in that**
a choice is made between the first and a second operating mode (M1, M2),
- wherein in the second operating mode (M2) only a 3D picture is generated for a plurality of all generated 2D images or generated sequences during a time interval (T) directly before and/or after a trigger command (A), and wherein the plurality of the generated 2D images or generated sequences or the generated 3D pictures are used to generate a general 3D picture.

2. The method according to claim 1, **characterised in that** the trigger command (A) is generated by actuating a foot switch.

3. The method according to claim 1, **characterised in that** the trigger command (A) is generated by a motion sensor when it detects that the mapping camera is stopped.

4. The method according to claim 1, **characterised in that** a shake number (V) is determined and compared with a limit value (G) during recording from at least two successively generated 2D images, and that a trigger command (A) is generated if the limit value (G) is not reached.

5. The method according to one of claims 1 through 4, **characterised in that** the 2D images or the sequences of 2D images are stored in succession in a buffer memory (8), a quantity of 2D images or sequences being stored, and after attainment of the quantity of already stored 2D images or sequences, they are overwritten by new 2D images or sequences.

6. The method according to one of claims 1 through 5, **characterised in that** after the trigger command (A), all generated 2D images or sequences of a plurality of 2D images within a time interval (T) preceding the trigger command (A) or within a time interval (T) following the trigger command (A) or within a time interval (A) comprising a point in time of the trigger command (A) are transferred to a memory.

7. The method according to one of claims 1 through 6, **characterised in that** the 2D images or additionally generated 2D images are displayed one after the other on a display means (5).

8. The method according one of claims 1 through 7, **characterised in that** the at least one 3D image is displayed on a display means (5).

9. The method according to one of claims 1 through 8, **characterised in that** the time interval (T) has a predetermined or predeterminable duration.

10. The method according to one of claims 1 through 8, **characterised in that** the time interval (T) begins with the trigger command (A) and ends after a stop command (S).

## Revendications

1. Procédé de mesure optique tridimensionnelle à l'aide d'une caméra (2) intraorale,
- ladite caméra (2) intraorale générant en continu dans une séquence temporelle des enregistrements 2D individuels ou des séquences d'une pluralité d'enregistrements 2D générés de manière successive,
- à partir d'au moins un des enregistrements 2D ou à partir d'au moins une des séquences, une image 3D étant générée et
- dans un premier mode de fonctionnement (M1), pour une pluralité de tous les enregistrements 2D générés ou de séquences générées, une image 3D étant générée et la pluralité d'enregistrements 2D générés ou de séquences générées ou les images 3D générées étant utilisées pour générer une image globale 3D,
**caractérisé en ce que**
le premier ou un second mode de fonctionnement (M1, M2) est choisi,
- dans le second mode de fonctionnement (M2), seulement pour une pluralité de tous les enregistrements 2D générés ou de séquences générées, pendant un intervalle de temps (T) directement avant et/ou après une commande de déclenchement (A), une image 3D étant générée et la pluralité d'enregistrements 2D générés ou de séquences générées ou les images 3D générées étant utilisées pour générer une image globale 3D.

2. Procédé selon la revendication 1, **caractérisé en ce que** la commande de déclenchement (A) est générée par actionnement d'un commutateur au pied.

3. Procédé selon la revendication 1, **caractérisé en ce que** la commande de déclenchement (A) est générée à l'aide d'un capteur de mouvement lorsque ledit capteur de mouvement détecte un état immobile de la caméra de mesure.

4. Procédé selon la revendication 1, **caractérisé en ce que**, pendant l'enregistrement, à partir d'au moins deux enregistrements 2D générés de manière successive, un nombre de flous d'image (V) est déterminé et comparé à une valeur limite (G) et que, lors du dépassement de la valeur limite (G), une commande de déclenchement (A) est générée.

5. Procédé selon l'une quelconque des revendications 1 à 4, **caractérisé en ce que** les enregistrements 2D ou les séquences d'enregistrements 2D sont mémorisés successivement dans une mémoire intermédiaire (8), une pluralité d'enregistrements ou de séquences 2D étant mémorisés et une fois le nombre atteint, les enregistrements ou les séquences 2D déjà mémorisés sont remplacés par de nouveaux enregistrements ou séquences 2D.

6. Procédé selon l'une quelconque des revendications 1 à 5, **caractérisé en ce que**, après la commande de déclenchement (A), tous les enregistrements 2D ou toutes les séquences de plusieurs images 2D générés dans un intervalle de temps (T) précédant la commande de déclenchement (A) ou tous les enregistrements 2D ou toutes les séquences de plusieurs images 2D générés dans un intervalle de temps (T) suivant la commande de déclenchement (A) ou tous les enregistrements 2D ou toutes les séquences de plusieurs images 2D générés dans un intervalle de temps (A) comprenant un instant de la commande de déclenchement (A) sont transférés dans une mémoire.

7. Procédé selon l'une quelconque des revendications 1 à 6, **caractérisé en ce que** les enregistrements 2D ou les enregistrements 2D générés en plus sont affichés successivement sur un moyen d'affichage (5).

8. Procédé selon l'une quelconque des revendications 1 à 7, **caractérisé en ce que** ledit enregistrement 3D est affiché sur un moyen d'affichage (5).

9. Procédé selon l'une quelconque des revendications 1 à 8, **caractérisé en ce que** l'intervalle de temps (T) présente une durée prédéfinie ou pouvant être prédéfinie.

10. Procédé selon l'une quelconque des revendications 1 à 8, **caractérisé en ce que** l'intervalle de temps (T) commence par la commande de déclenchement (A) et se termine après une commande d'arrêt (S).
